# EUROPEAN PATENT APPLICATION

(11) **EP 2 567 678 A1**
(43) Date of publication of application: **13.03.2013**
(21) Application number: 12007781.3
(22) Date of filing: 16.05.2005
(51) Int. Cl.: A61F 2/915, A61F 2/00

(54) **Stent with contoured bridging elements**

(30) Priority: 21.05.2004 US 573085 P
(62) Divisional of application: 05749784.4
(71) Applicant: Conor Medsystems, Inc., Menlo Park, CA 94025 (US)
(72) Inventor: Shanley, John F., Emerald Hills, CA 94062-4072 (US)
(74) Representative: Diehl & Partner GbR

(57) **Abstract**

A stent (10) comprising: a plurality of expandable rings (20) formed of a plurality of struts; and a plurality of flexible bridging elements (30)interconnecting the plurality of expandable rings, which allow the stent to flex axially, the plurality of flexible bridging elements including at least one flex member (32), wherein the at least one flex member is contoured by varying a width of the flex member continuously along its length over an arc of at least 180°.

## Description

### RELATED APPLICATIONS

This application claims priority to U.S. Provisional Patent Application Serial No. 60/573,085, filed May 21, 2004, the entire contents of which are incorporated herein by reference.

### Background

Stents are expandable implantable devices which are adapted to be implanted into a patient's body to maintain patency of a body lumen. When used in blood vessels, stents can serve to prevent vessels from collapsing, reinforce vessel walls, increase cross sectional area, increase blood flow, and restore or maintain healthy blood flow. Early stent structures included simple wire meshes or coils which were expanded radially outward within a lumen of the human body to support the lumen.

Examples of early stents are described in U.S. Patent Nos. 4,733,665; 5,102,417; 5,421,955; and 5,902,332.

One method often used for delivery and implantation of stents employs an expandable member, such as a balloon catheter to deliver the stent to a desired location within the patient's body and to expand the stent into an expanded implanted configuration. One of the difficulties in delivery of stents to an implantation site within the body is navigation of the often tortuous path of the vasculature.

Stents have been designed with flexible bridging elements between relatively rigid cylindrical sections. The flexible bridging elements allow the stent to flex axially during delivery and upon implantation. Examples of flexible bridging elements are shown in U.S. Patent Nos. 5,449,373; 5,697,971; and 6,241,762. The use of multiple bends in a bridging element has been shown to provide good flexibility.

Practitioners are always in search of a more flexible and thus more deliverable stent. Meanwhile, stent designs are limited by the practical requirements for radial hoop strength, longitudinal dimensional stability, fatigue strength, and coverage area.

### Summary of the Invention

The present invention relates to a stent having flexible bridging elements which are contoured along their length to uniformly distribute energy during deformation.

In accordance with one aspect of the invention, a stent includes a plurality of expandable rings formed of a plurality of struts; and a plurality of flexible bridging elements interconnecting the plurality of expandable rings and allowing the stent to flex axially. The plurality of flexible bridging elements include at least two curved flex members which are contoured by varying their cross sections along their length to distribute strain substantially uniformly along the curved flex members.

In accordance with another aspect of the invention, a stent includes a plurality of expandable rings; and a plurality of bridging elements interconnecting the rings, the bridging elements including at least one curved flex member having a gradually tapering width throughout with a width at a center portion of the at least one curved flex member which is larger than a width at opposite two end portions of the at least one curved flex member. The at least one curved flex member has an average radius of curvature of at least two times a largest width of the at least two curved flex members.

In accordance with an additional aspect of the invention, a stent includes a plurality of expandable rings formed of a plurality of struts; and a plurality of flexible bridging elements interconnecting the plurality of expandable rings and allowing the stent to flex axially. The plurality of flexible bridging elements include at least one flex member which is contoured by varying a width of the flex member continuously along its length in an arrangement which distributes strain substantially uniformly along the at least one flex member.

### Brief Description of the Drawings

The invention will now be described in greater detail with reference to the preferred embodiments illustrated in the accompanying drawings, in which like elements bear like reference numerals, and wherein:
FIG. 1A is an enlarged perspective view of one example of a stent according to the present invention in a semi expanded configuration.
FIG. 1B is a top view of the stent of FIG. 1A which has been unrolled and laid flat.
FIG. 2 is an enlarged view of a bridging element of an uncontoured design.
FIG. 3 is a Goodman Diagram of the FIG. 2 design.
FIG. 4 is an enlarged view of a bridging element of a contoured design.
FIG. 5 is a Goodman Diagram of the FIG. 4 design.
FIG. 6 is an enlarged view of a bridging element of the stent of FIGS. 1A and 1B.
FIG. 7 is a Goodman Diagram of the FIG. 6 design.

### Detailed Description

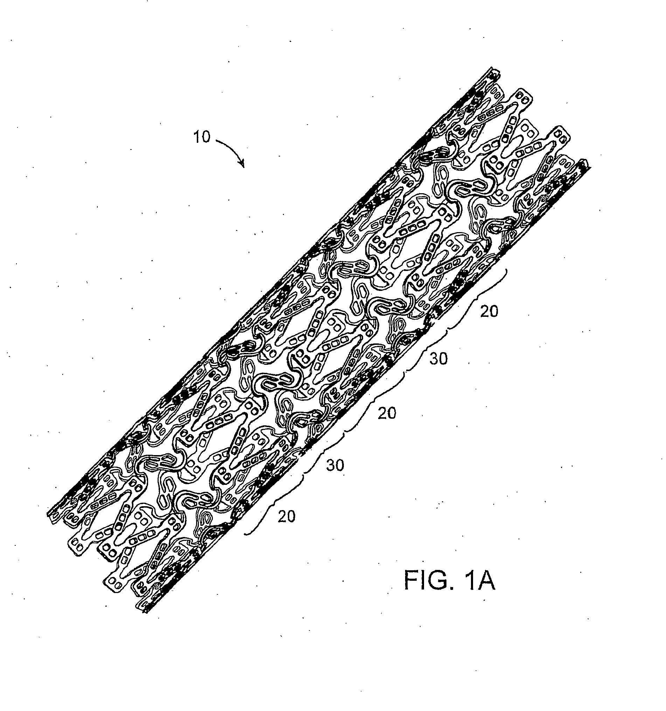

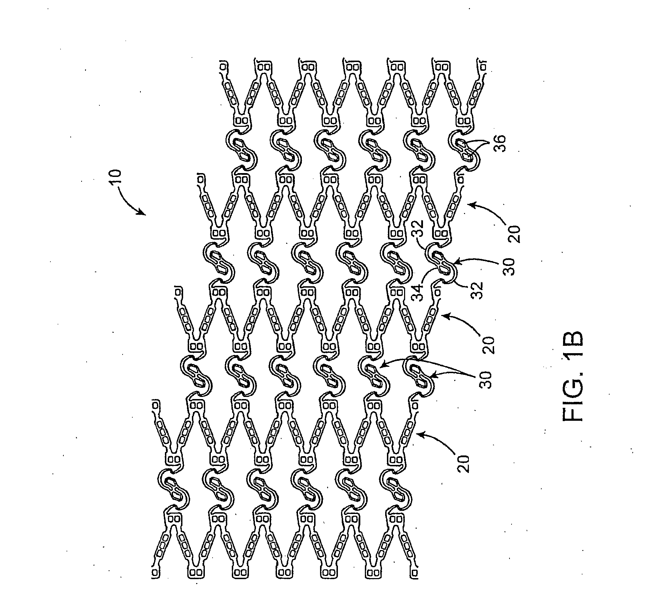

FIGS. 1A and 1B illustrate a stent 10 formed from a plurality of expandable rings 20 and a plurality of flexible bridging elements 30 connecting the rings. The stent 10 is expandable from an insertion configuration to an expanded implanted configuration by deployment of an expanding device, such as a balloon catheter. The expandable rings 20 provide radial hoop strength to the stent while the flexible bridging elements 30 allow the stent to flex axially during delivery and upon implantation. The flexible bridging elements 30 are designed with elements having varying widths contoured to distribute strain substantially uniformly along the bridging elements. The contoured shapes of the bridging elements 30 maximizes fatigue strength and flexibility of the bridging elements.

The term "width" as used herein means a dimension of an element in a plane of the cylindrical surface of the stent. The width is generally measured along a line substantially perpendicular to the edges of the element.

In the embodiment illustrated in FIGS. 1A and 1B, the expandable rings are formed by a plurality of struts 22 and a plurality of ductile hinges 24 arranged such that upon expansion, the ductile hinges are deformed while the struts are not substantially deformed. The ductile hinge 24 and strut 22 structures are described in further detail in U.S. Patent No. 6,241,762 which is incorporated herein by reference in its entirety. As shown in FIGS. 1A and 1B, the rings 20 have alternating open and closed ends. In the arrangement of FIGS. 1A and 1B, the closed ends of the rings 20 are aligned with closed ends of adjacent rings and the closed ends are interconnected by the flexible bridging elements 30. The adjacent closed ends can be referred to as structures which are substantially 180° out of phase. The expandable rings may alternatively be formed in any of the other known ring structures including serpentine rings, diamond structures, chevron shapes, or the like which are in phase or out of phase.

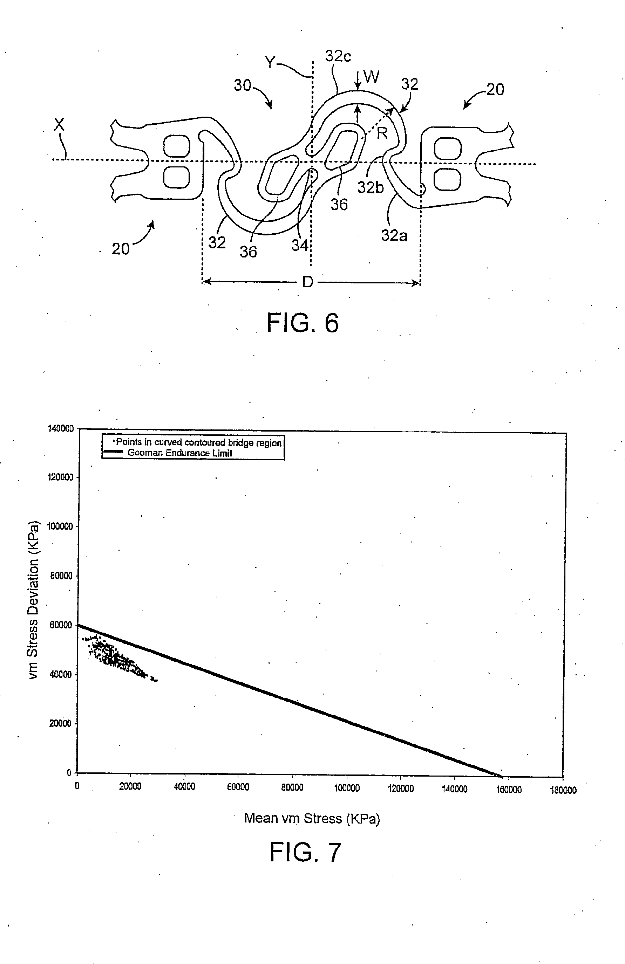

The flexible bridging elements 30 of the stent 10 of FIGS. 1A and 1B have been designed to maximize fatigue strength and flexibility. An enlarged view of one of the flexible bridging elements 30 of FIGS. 1A and 1B is shown in FIG. 6. The bridging element 30 includes two curved flex members 32 each connecting an adjacent expandable ring 20 to a central reservoir containing structure 34. The least two curved flex members 32 are contoured by varying their cross sections along their length to distribute strain substantially uniformly along the curved flex members.

The central reservoir containing structure 34 may take on many different shapes depending on the space available and the amount of drug to be delivered from the reservoirs. In the example of FIGS. 1A and 1B, the reservoir containing structure 34 includes two irregular polygonal holes 36 arranged partially within the arch shapes formed by the curved flex members 32.

The reservoir containing structure 34 can support one, two or more reservoirs which can be non-deforming or substantially non-deforming. The reservoir containing structures 34 allow the stent to deliver one or more beneficial agents which can be delivered luminally, murally, or bi-directionally. The use of a reservoir containing structure 34 within the bridging elements 30 allows a beneficial agent to be distributed more evenly along the length of the stent. However, the reservoir containing structures 34 can also be omitted from the bridging elements 30.

The term "beneficial agent" as used herein is intended to have its broadest possible interpretation and is used to include any therapeutic agent or drug, as well as inactive agents such as barrier layers, carrier layers, therapeutic layers, or protective layers. Exemplary classes of therapeutic agents which can be used in the beneficial agent of the present invention include one or more antiproliferatives (paclitaxel and rapamycin), antithrombins (i.e., thrombolytics), immunosuppressants, antilipid agents, anti-inflammatory agents, antineoplastics, antimetabolites, antiplatelets, angiogenic agents, anti-angiogenic agents, vitamins, antimitotics, NO donors, nitric oxide release stimulators, anti-sclerosing agents, vasoactive agents, endothelial growth factors, insulin, insulin growth factors, antioxidants, membrane stabilizing agents, and anti-restenotics.

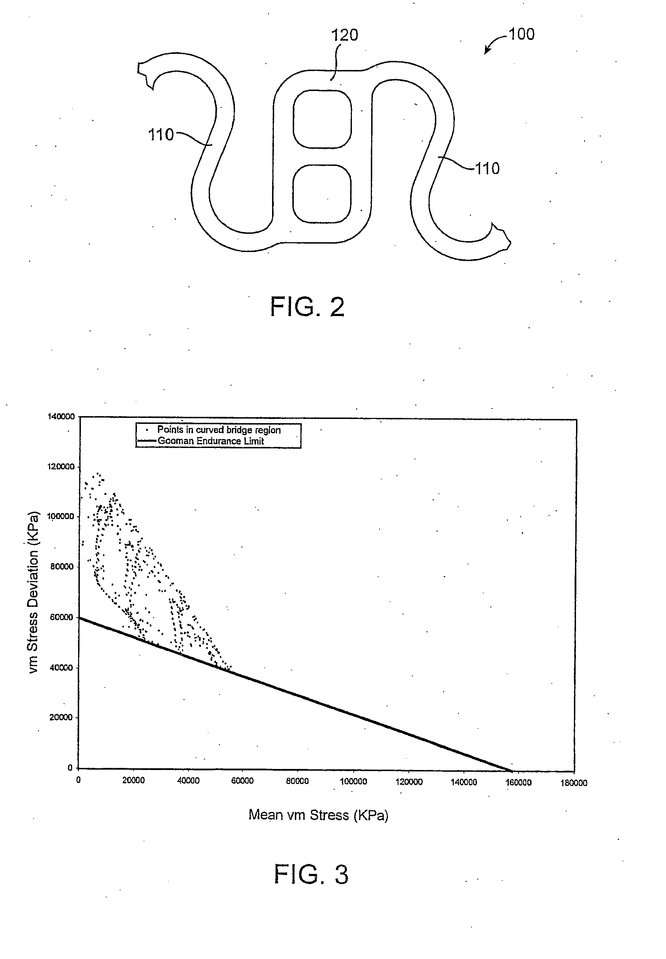

FIGS. 2-7 illustrate the improved fatigue strength of the bridging elements achieved by contouring the bridging elements to uniformly distribute energy. This improved fatigue strength provided by contoured bridging elements is illustrated most clearly by the Goodman Diagrams of FIGS. 3, 5, and 7 associated with designs having differing degrees of contouring in the bridging elements.

The most common method of assessing the fatigue characteristics of a ferrous metal structure is to construct a Goodman Diagram. A Goodman Diagram is generated as an X-Y graph where the X-axis is "mean stress" or average stress, also called the steady state stress. The Y-axis is the "alternating stress" or cyclic stress, also called the stress deviation. A Goodman Line is constructed by connecting a point on the X-axis at the tensile strength of the material with a point on the Y-axis that is at the endurance limit of the material. For a plurality of locations on the structure to be analyzed a point is plotted on the Goodman Diagram. If the point lies above the Goodman Line the structure will eventually fail at this location. If the point is below the Goodman Line then the structure will have infinite life at that location. As you increase either the mean stress or the cyclic stress, the point will eventually move above the Goodman Line.

In order to create a Goodman Diagram for a stent bridge structure of a stent, the stent structure is analyzed to determine the average amount of initial and cyclic deformation experienced by a single bridge structure when the stent is expanded. Both initial deformation during deployment and cyclic deformation due to beating of the heart are established to provide the parameters for structural analysis. To determine initial deformation of a bridge structure the actual deformation or elongation of the bridging elements upon expansion of the stent can be measured or calculated. The cyclic deformation of the implanted stent can be calculated based on the known physiological characteristics of the heart. These two deformations, the initial expansion deformation and the cyclic deformation, are applied to the structure is structural analysis to determine the mean stress and stress deviation for a plurality of points on the structure.

FIG. 2 illustrates a bridging element 100 having two S-shaped links 110 and a central reservoir containing structure 120. The two S-shaped links 110 are uncontoured (uniform in cross section) resulting in areas of peak strain at the inner side of the curved portions. FIG. 3 shows the Goodman Diagram of such a structure formed of a cobalt chromium alloy such as standard "L605" (ASTM F 90, ISO 5832-5) cobalt chromium alloy. This alloy has the composition Co, 20 wt.% Cr, 15 wt.% W, 10 wt.% Ni and 1.5 wt.% Mn. As shown in FIG. 3, in the uncontoured S-shaped design most of the points in the curved bridge regions or S-shaped links 110 are located on the Goodman Diagram above the Goodman Line. FIG. 3 also illustrates a large variation in the mean stresses and stress deviations experienced by the different points in the curved bridge which results from the uncontoured structure. For example, the stress deviation varies between about 40,000 and about 120,000 KPa (about 80,000 KPa) and the mean stress goes up to about 55,000 KPa.

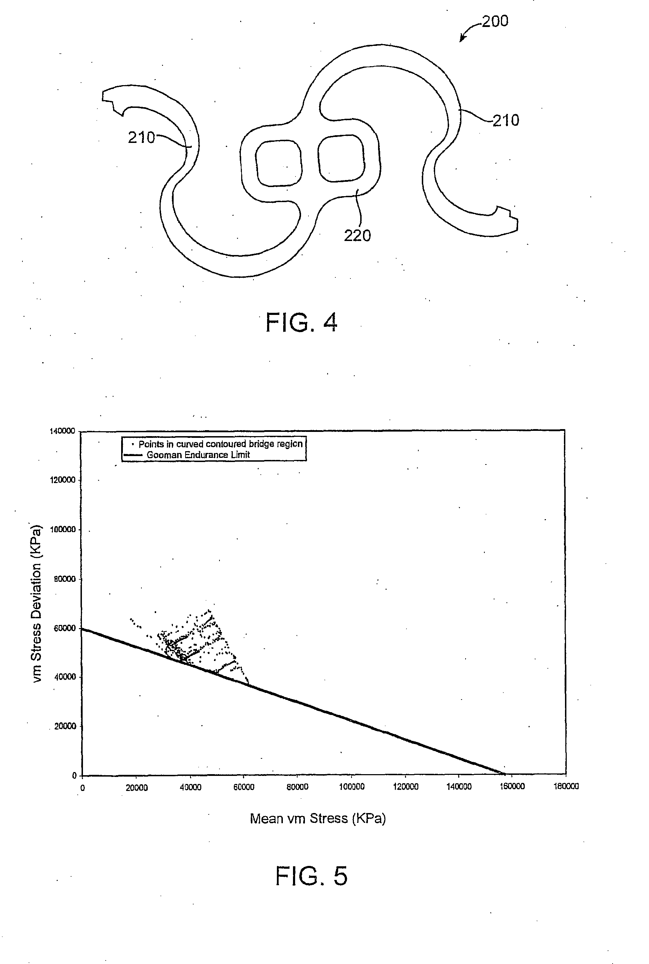

FIG. 4 illustrates a bridging element 200 with a contoured design of S-shaped links 210 and a central reservoir containing structure 220. The use of contouring or changes in the width of the S-shaped links 210 has distributed the strain more uniformly along the curved members. As shown in the Goodman Diagram of FIG. 5, the contoured bridging element of FIG. 4 provides a significant improvement over the FIG. 2 design in bringing the points in the S-shaped bridge links 210 closer to the Goodman Line and particularly in grouping the points closer together.

The use of the contoured bridging elements 210 of FIG. 4 has caused the points in FIG. 5 to be grouped more closely together indicating that the energy is more evenly distributed in the structure. In FIG. 5, the variation between the points with the highest and lowest stresses is significantly reduced from the uncontoured example of FIG. 2. Specifically, the stress deviation varies between about 40,000 and about 70,000 KPa. A total variation from maximum to minimum stress deviation is about 30,000 KPa. The mean stress varies from about 20,000 KPa to about 60,000 KPa (a variation of about 40,000 KPa).

FIG. 6 illustrates an enlarged view of one of the bridging elements 30 of FIGS.
1A and 1B with a contoured design. The use of a varying width throughout the bridging element curved flex members 32 has distributed the strain uniformly throughout the structure. As shown in the Goodman Diagram of FIG. 7, the contoured bridging element 30 of FIG. 6 shows all the points in the bridging element are clustered closely together and located below the Goodman Line. A total variation from maximum to minimum stress deviation is less than about 20,000 KPa and a variation of mean stress is about 30,000 KPa. Variations in the structure of the stent, such as larger cylindrical segments and fewer bridging elements, can cause the Goodman Diagram to change by moving the locations of the points on the graph, however the close grouping of the points is created by the finely tuned contouring of the bridging elements. Thus, the present invention is useful in any stent having a curved bridging structure which can be contoured to group points in the Goodman Diagram close together.

The curved flex members 32 of FIG. 6 each include a connection leg 32a, an offset leg 32b and an arc shaped leg 32c. The arc shaped legs 32c are connected to the central reservoir containing structure 34 and pass over a circumferentially oriented centerline Y of the bridging element 30. The arc shaped legs 32c have been made larger by passing over the centerline Y and by the offset leg 32b to increase the amount of material which is available to store energy during oscillation.

The contours of the arc shaped legs 32c have continuously changing widths and radii of curvature which have been selected based on structural analysis of the forces in the legs. Thus, the arc shaped legs 32c are each asymmetrical. The two arc shaped legs 32c in each bridging element 30 are generally inverted mirror images of one another. Although the radius of curvature of the enlarged arc shaped legs 32c varies somewhat along a length of the arc shaped legs, the average radius of curvature R is preferably at least 15% of an axial distance D between the plurality of expandable rings. In addition, the arc shaped legs 32c have an average radius of curvature R of at least 2 times a largest width W of the at least two curved flex members. This largest width W of the arc shaped legs 32c is located at a center portion of the arc shaped legs and is larger than a width at the opposite two end portions of the arc. Thus, the portions of the arc shaped legs 32a farthest from an axial centerline X of the bridging elements 30 have the largest widths to substantially eliminate the concentration of forces occurring at these points. The arc shaped legs 32c are continuously curving without a distinct point of inflection.

In the embodiment of FIG. 6, the connection legs 32a are connected to the rings 20 above and below an axial centerline X extending through the bridging element 30. However, the connection legs 32a can also be connected at other locations such as, along the centerline X, on the struts 22, both above, or both below the centerline X. The connection leg 32a can also be contoured with a continuously changing width to uniformly distribute energy in this portion of the bridging element 30.

In the embodiment of FIG. 6, the contouring of the arc shaped legs 32c and the connection legs 32a results in a structure in which a largest width W of the curved flex members 32 is at least about 1.5 times a smallest width of the curved flex members. Preferably, the largest width W is about 2 times the smallest width.

The particular contours of the bridging elements will vary depending on the stent material and the particular bridge design. However, in general, the portion of the bridge with the largest width will be located farthest from the centerline X extending between cylindrical elements. More particularly, the largest width will be located approximately at the locations which are the farthest from a line intersecting connection points between the bridging elements and the cylindrical elements. The changes in width of the bridging elements 30 are gradual throughout the structure to avoid any concentration of forces. The contoured bridging elements 30 provide a structure in which elastic deformation is distributed evenly in the contoured portions.

Although the present invention has been described to involve contouring of the bridging elements by varying the width of the bridging elements along their length, the contouring can also be achieved by varying a thickness of the bridging elements, or by varying both width and thickness.

The particular contours and dimensions of the bridging elements 30 can vary depending on the stent structure and material. The bridging elements 30 have been designed for a stent formed of a cobalt chromium alloy. Other materials from which the stent can be made include stainless steel, other metal alloys, polymers, or biodegradable polymers or metal alloys.

While the invention has been described in detail with reference to the preferred embodiments thereof, it will be apparent to one skilled in the art that various changes and modifications can be made and equivalents employed, without departing from the present invention.

In summary, the subject matter described in the following paragraphs, which are numbered to allow easy reference, is part of the disclosure of the present application, each of which can be claimed in the present application, and in one or more future divisional applications there from:
(1) A stent comprising: a plurality of expandable rings formed of a plurality of struts; and plurality of flexible bridging elements interconnecting the plurality of expandable rings and allowing the stent to flex axially, the plurality of flexible bridging elements including at least two curved flex members, wherein the at least two curved flex members are contoured by varying their cross sections along their length to distribute strain substantially uniformly along the curved flex members.
(2) The stent of paragraph 1, wherein the plurality of bridging elements include at least one reservoir containing a beneficial agent.
(3) The stent of paragraph 2, wherein the at least one reservoir is positioned between the at least two curved flex members in an enlarged portion of the plurality of bridging elements.
(4) The stent of paragraph 1, wherein the plurality of bridging elements include an enlarged portion having a width larger than a largest width of the at least two curved flex members, the enlarged portion including at least one reservoir containing a beneficial agent.
(5) The stent of paragraph 1, wherein the at least two curved flex members have a largest width at a central portion and taper to smaller widths at opposite ends.
(6) The stent of paragraph 1, wherein widest portions of the at least two curved flex members are located at portions of the plurality of bridging elements which are farthest from an axially oriented center line of the plurality of bridging elements.
(7) The stent of paragraph 1, wherein the plurality of expandable rings are each formed from a plurality of struts which open into substantially V or U shapes.
(8) The stent of paragraph 1, wherein adjacent ones of the plurality of expandable rings are out of phase.
(9) The stent of paragraph 1, wherein the at least two curved flex members each have a radius of curvature of at least 15% of an axial distance between the plurality of expandable rings.
(10) The stent of paragraph 1, wherein the at least two curved flex members have a radius of curvature of at least 2 times a largest width of the at least two curved flex members.
(11) The stent of paragraph 1 , wherein an elastic deformation of the flexible bridging elements is distributed evenly along the at least two curved flex members when the stent is expanded from an insertion diameter to an implanted diameter.
(12) The stent of paragraph 1, wherein the at least two curved flex members include have a continuously curving surface without points of inflection.
(13) The stent of paragraph 1, wherein the at least two curved flex members are asymmetrical.
(14) The stent of paragraph 1, wherein a rigid member interconnects the at least two curved flex members.
(15) A stent comprising: a plurality of expandable rings; and a plurality of bridging elements interconnecting the rings, the bridging elements including at least one curved flex member having a gradually tapering width throughout with a width at a center portion of the at least one curved flex member which is larger than a width at opposite two end portions of the at least one curved flex member, the at least one curved flex member having an average radius of curvature of at least two times a largest width of the at least two curved flex members.
(16) The stent of paragraph 15, wherein the plurality of bridging elements include at least one reservoir containing a beneficial agent.
(17) The stent of paragraph 15, wherein the plurality of expandable rings are each formed from a plurality of struts which open into substantially V or U shapes.
(18) The stent of paragraph 15, wherein the at least two curved flex members each have a radius of curvature of at least 15% of an axial distance between the plurality of expandable rings.
(19) The stent of paragraph 15, wherein the at least two curved flex members are asymmetrical.
(20) A stent comprising: a plurality of expandable rings formed of a plurality of struts; and a plurality of flexible bridging elements interconnecting the plurality of expandable rings and allowing the stent to flex axially, the plurality of flexible bridging elements including at least one flex member, wherein the at least one flex member is contoured by varying a width of the flex member continuously along its length in an arrangement which distributes strain substantially uniformly along the at least one flex member.
(21) The stent of paragraph 20, wherein the plurality of bridging elements include at least one reservoir containing a beneficial agent.
(22) The stent of paragraph 20, wherein the plurality of expandable rings are each formed from a plurality of struts which open into substantially V or U shapes.
(23) The stent of paragraph 20, wherein the at least two curved flex members each have a radius of curvature of at least 15% of an axial distance between the plurality of expandable rings.
(24) The stent of paragraph 20, wherein the at least two curved flex members are asymmetrical.
(25) The stent of paragraph 20, wherein the contouring of the at least one flex member is configured to achieve a total variation from maximum to minimum stress deviation of points distributed along the structure of less than about 30,000 KPa and a variation of mean stress of the points of less than about 40,000 KPa.
(26) The stent of paragraph 20, wherein the contouring of the at least one flex member is configured to achieve a total variation from maximum to minimum stress deviation of points distributed along the structure of less than about 20,000 KPa and a variation of mean stress of the points of less than about 30,000 KPa.

## Claims

1. A stent comprising:
a plurality of expandable rings formed of a plurality of struts; and
a plurality of flexible bridging elements interconnecting the plurality of expandable rings and allowing the stent to flex axially, the plurality of flexible bridging elements including at least one flex member, wherein the at least one flex member is contoured by varying a width of the flex member continuously along its length over an arc of at least 180°.

2. The stent of Claim 1, wherein the plurality of bridging elements include at least one reservoir containing a beneficial agent.

3. The stent of Claim 1, wherein the plurality of expandable rings are each formed from a plurality of struts which open into substantially V or U shapes.

4. The stent of Claim l, wherein the at least two curved flex members each have a radius of curvature of at least 15% of an axial distance between the plurality of expandable rings.

5. The stent of Claim 1, wherein the at least two curved flex members are asymmetrical.

6. The stent of Claim 1, wherein the contouring of the at least one flex member is configured to achieve a total variation from maximum to minimum stress deviation of points distributed along the structure of less than about 20,000 KPa and a variation of mean stress of the points of less than about 30,000 KPa.

7. A method for preparing a stent, which method comprises:
providing a stent with a plurality of expandable rings formed of a plurality of struts and a plurality of flexible bridging elements interconnecting the plurality of expandable rings which allow the stent to flex axially, the plurality of flexible bridging elements including at least one flex member, wherein the at least one flex member is contoured by varying a width of the flex member continuously along its length in an arrangement which distributes strain more uniformly along the at least one flex member than in an uncontoured curved flex member having a uniform cross section,
wherein the contouring of the at least one flex member is configured to achieve a total variation from maximum to minimum stress deviation of points distributed along the structure of less than about 30,000 KPa and a variation of mean stress of the points of less than about 40,000 KPa.
